# EUROPEAN PATENT APPLICATION

(11) **EP 1 541 140 A1**
(43) Date of publication of application: **15.06.2005**
(21) Application number: 03788067.1
(22) Date of filing: 08.08.2003
(51) Int. Cl.: A61K 31/198, A61K 9/20, A61K 47/10, A61K 47/26, A61K 47/36, A61K 47/38

(54) **AMINO ACID-CONTAINING CHEWABLE**

(30) Priority: 12.08.2002 JP 2002235160; 25.12.2002 JP 2002375397
(71) Applicant: KYOWA HAKKO KOGYO CO., LTD., Chiyoda-ku, Tokyo 100-8185 (JP)
(72) Inventor: OTA, Motohiro, c/o Pharm. Res. Inst. Kyowa Hakko, Sunto-gun, Shizuoka 411-8731 (JP); MORIMOTO, Kiyoshi, Pharm. Res. Inst. Kyuowa Hakko, Sunto-gun, Shizuoka 411-8731 (JP); KUBOYAMA, Muneko, c/o Head Office Kyowa, Chiyoda-ku, Tokyo 100-8185 (JP); YOSHIMOTO, Hirokazu, Pharm. Res. Inst. Kyowa Hakko, Sunto-gun, Shizuoka 411-8731 (JP); KAWABE, Hideo, c/o Head Office, Chiyoda-ku, Tokyo 100-8185 (JP); SHIBASAKI, Takeshi, c/o Head Office, Chiyoda-ku, Tokyo 100-8185 (JP)
(74) Representative: VOSSIUS & PARTNER
(86) International application number: PCT/JP2003/010181
(87) International publication number: WO 2004/016262

(57) **Abstract**

The present invention provides a chewable tablet having an improved oral disintegration property, which is easily chewed and capable of containing a large amount of an amino acid depending on need, more specifically, a chewable tablet comprising an amino acid and an oral disintegration promoting agent.

## Description

### Technical Field

The present invention relates to chewable tablets comprising an amino acid. In more detail, the present invention relates to an amino acid-containing chewable tablet having an improved oral disintegration property, and a method for manufacturing the same.

### Background Art

The muscle fatigue-reducing effect and the fatty acid burning effect of nutritious food containing amino acid as a main ingredient, have been appreciated by consumers, and demands for amino acid-containing nutritious food for sports, dieting, etc. has been sharply increasing. In order to suit such purposes, essential requirements for an ideal amino acid-containing nutritious food is small, portable and easy to take outdoors, and that it is possible to take a large amount of an amino acid at a time. Among the conventional amino acid-containing nutritious foods, however, a drink, a jelly, etc. are inconvenient to carry around, and a granule, a conventional type of a tablet, etc. are inconvenient for taking because they should be taken with water. Further, since a conventional type of a tablet has a limitation on the tablet diameter, it is not possible to take a large amount of an amino acid. Thus, any of the currently available amino acid-containing nutritious food does not satisfy the requirements which are ideal for a commercial product.

With regard to an amino acid-containing chewable tablet by which an amino acid can be taken without water, there is a known chewable tablet containing an amino acid at 20 to 30% by weight of the tablet, which is prepared using hydrogenated maltose or dextrin as an excipient. With this level of amino acid content, however, it is difficult to take a large amount of an amino acid at a time.

As for an amino acid preparation for sports, it is preferable that a large amount of an amino acid which is functional during exercises can be quickly taken. However, a conventional amino acid-containing chewable tablet has disadvantages; it is difficult to take the tablet during exercises because it requires large force to chew the tablet and unpleasant feeling remains in the oral cavity after chewing, and it is not possible to contain sufficient amount of amino acid in the tablet to appropriately function for the purpose of sports due to its poor molding property for tableting. For example, the average amino acid content in an amino acid granule for sports is about 2 to 3 grams for a pack, while that of the conventional chewable tablet is as small as about 0.2 to 0.3 grams even in a large-sized tablet having the tablet weight of about 1 g, which indicates that it is substantially difficult to take an amino acid for the sports by tablets.

On the other hand, among the conventional arts for pharmaceutical preparations, there are common tablets which are dissolved in a stomach and rapidly disintegrable tablets which comprise a disintegration promoting agent so that they are rapidly disintegrated in a mouth without chewing (see, for example, Japanese Published Unexamined Patent Application Nos. 271,054/93 and 182,436/98). However, there has been no example that a disintegration promoting agent is added to a chewable tablet which is to be disintegrated by chewing in the oral cavity.

### Disclosure of the Invention

An object of the present invention is to provide an amino acid-containing chewable tablet which is easily chewed and can contain a large amount of an amino acid depending on need.

In order to solve the problem, the present inventors have carried out intensive investigations and, as a result, they have found that, when the oral disintegration property of an amino acid-containing tablet is improved, it is possible to provide a soft chewable tablet which can be chewed by a weak force even during vigorous exercises and gives no unpleasant feeling remained, and that the chewable tablet can contain a large amount of an amino acid. The present inventors have conducted further studies, and finally completed the present invention.

Thus, the present invention relates to:
(1) A chewable tablet comprising an amino acid and having an improved oral disintegration property;
(2) An intraorally rapidly disintegrable chewable tablet comprising an amino acid;
(3) The chewable tablet according to the above (1) or (2), which comprises an oral disintegration promoting agent;
(4) The chewable tablet according to any one of the above (1) to (3), which further comprises a saccharide and/or a sugar alcohol;
(5) A chewable tablet comprising an amino acid and an oral disintegration promoting agent;
(6) The chewable tablet according to the above (5), which further comprises a saccharide and/or a sugar alcohol;
(7) The chewable tablet according to any one of the above (2) to (6), wherein the oral disintegration promoting agent is sodium starch glycolate or calcium carboxymethylcellulose;
(8) The chewable tablet according to any one of the above (1) to (7), wherein the time until one tablet is disintegrated by saliva alone after chewing in the oral cavity of a healthy adult is 60 to 150 seconds;
(9) The chewable tablet according to any one of the above (1) to (8), wherein the amino acid content is 30 to 85% by weight;
(10) The chewable tablet according to any one of the above (1) to (9), wherein the hardness of the tablet is 60 N or more;
(11) The chewable tablet according to any one of the above (1) to (10), wherein the amino acid comprises one or, two or more members selected from the group consisting of valine, leucine and isoleucine;
(12) The chewable tablet according to any one of the above (1) to (11), wherein the amino acid is a mixture of a pure amino acid and a proteolytic mixture;
(13) A method for manufacturing a chewable tablet having an improved oral disintegration property, which comprises subjecting powder particles comprising an amino acid and an oral disintegration promoting agent to compression molding;
(14) A method for manufacturing an intraorally rapidly disintegrable chewable tablet, which comprises subjecting powder particles comprising an amino acid and an oral disintegration promoting agent to compression molding;
(15) The method for manufacturing a chewable tablet according to the above (13) or (14), wherein the powder particles further comprise a saccharide and/or a sugar alcohol;
(16) A portable package in which the chewable tablet according to any one of the above (1) to (12) is contained; and
(17) The portable package according to the above (16), wherein the chewable tablet is contained together with a desiccant.

### Best Mode for Carrying Out the Invention

An amino acid used in the present invention may be one or, a combination of two or more pure amino acids prepared by fermentation, synthesis or extraction from a plant, and may be a proteolytic mixture such as whey protein, soybean protein, etc., and also may be an appropriate combination of a pure amino acid with a proteolytic mixture.

Examples of the pure amino acid in the present invention are aliphatic amino acids such as glycine and alanine; branched-chain amino acids such as valine, leucine and isoleucine; hydroxyamino acids such as serine and threonine; acidic amino acids such as aspartic acid and glutamic acid; amides such as asparagine and glutamine; basic amino acids such as lysine, hydroxylysine, arginine and ornithine; sulfur-containing amino acids such as cysteine, cystine and methionine; aromatic amino acids such as phenylalanine and tyrosine; heterocyclic amino acids such as tryptophane and histidine; cyclic amino acids such as proline and 4-hydroxyproline and the like, and it is preferable that one or, two or more members of the aforementioned amino acids, a derivative thereof, etc. is/are contained in the chewable tablet of the present invention. Examples of the derivative of an amino acid are acetylglutamine, acetylcysteine, carboxymethylcysteine, acetyltyrosine, acetylhydroxyproline, 5-hydroxyproline, glutathione, creatine, S-adenylmethionine, glycylglycine, glycylglutamine, DOPA (dihydroxyphenylalanine), alanylglutamine, carnitine, etc.

The amino acid used in the present invention may be a salt, and examples of such a salt are salts with an organic or inorganic acid such as hydrochloride, sulfate and acetate and salts with a base such as sodium salt and potassium salt, and the like.

In the present invention, the amino acid is preferably one or, a combination of two members selected from branched amino acids such as valine, leucine and isoleucine; amides such as asparagine and glutamine; basic amino acids such as lysine, hydroxylysine and arginine; and cyclic amino acids such as proline, more preferably one or, a combination of two or more members selected from branched amino acids such as valine, leucine and isoleucine and amides such as asparagine and glutamine.

With regard to one or, combination of two or more amino acid(s), an example thereof includes a combination of three branched amino acids comprising, for example, valine, leucine and isoleucine (hereinafter, referred to as a composition combining three branched amino acids), and examples of the composition combining three branched amino acids preferably include those in which the ratio of valine, leucine and isoleucine is (0.8 to 1.2) : (1.3 to 2.5) : (0.8 to 1.2). There may be exemplified a composition in which glutamine is used alone or in combination with one or, two or more members of the aforementioned branched amino acid(s), to be more specific, a composition in which glutamine is compounded with the aforementioned composition combining three branched amino acids at 0.1- to 5-fold the amount of the composition combining three branched amino acids. Also, a composition in which arginine is used alone or in combination with glutamine or in combination with glutamine and one or, two or more members of the aforementioned branched amino acid(s); a composition in which arginine is combined with one or, two or more members of the aforementioned branched amino acid(s) and the like may be exemplified. To be more specific, with the aforementioned composition combining three branched amino acids and composition in which glutamine added to at a ratio of 1:(0.1 to 5), further a composition in which 0.1- to 5-fold of arginine is added to the aforementioned composition combining three branched amino acids, and a composition in which 0.1- to 5-fold of arginine is compounded with the aforementioned composition combining three branched amino acids may be exemplified.

It is preferable that the chewable tablet comprising an amino acid and having an improved oral disintegration property contains an amino acid at about 1 to 85% by weight, more preferably 35 to 80% by weight. In order to achieve a high function for the use of an amino acid as nutrition, an amino acid may be contained preferably about 30 to 85% by weight, more preferably about 35 to 70% by weight and particularly preferably 40 to 65% by weight.

Although there is no particular limitation on the average particle size of the amino acid, it is preferably 200 µm or smaller, more preferably 100 µm or smaller and particularly preferably 50 µm or smaller.

Improvement in an oral disintegration property according to the present invention means, for example, that the time needed for disintegration of the tablet in the oral cavity without chewing but by saliva alone or the time needed for disintegration of the tablet in the oral cavity after chewing is shortened as compared to the case of the conventional chewable tablets, while a rapid oral disintegration property means, for example, the property in which the time needed for disintegration of the tablet in the oral cavity without chewing by saliva alone or the time needed for disintegration of the tablet in the oral cavity after chewing is shortened as compared to the case of the conventional chewable tablets, and improvement in taking property should be achieved. As a result of such improvements, it is now possible to increase the amount of an amino acid in the tablet, whereby the tablet size becomes smaller and the taking property can be enhanced. An example of the means for improving the disintegration property in the oral cavity or the means for giving the oral disintegration property is a method in which an oral disintegration promoting agent is added to the tablet, and the like.

Examples of the oral disintegration promoting agent in the present invention are carboxymethylcellulose, calcium carboxymethylcellulose, sodium carboxymethylcellulose, crospovidone, sodium croscarmellose, sodium starch glycolate and the like. It is preferred to use calcium carboxymethylcellulose or sodium starch glycolate, and it is particularly preferred to use carboxymethylcellulose either alone or in combination with others.

Such oral disintegration promoting agents are preferably used only by mixing so that they do not coat the surface of the amino acid particles or amino acid-containing granules.

In the present invention, the oral disintegration promoting agents can be contained, either alone or in combination, in an amount of preferably about 0.5 to 20% by weight, more preferably about 0.5 to 5% by weight and, still more preferably, about 0.5 to 2% by weight of the tablet.

Although there is no particular limitation on the average particle size of the oral disintegration promoting agent, it is preferably 200 µm or smaller, more preferably 100 µm or smaller and particularly preferably 50 µm or smaller.

In the present invention, examples of the saccharide are a monosaccharide, a disaccharide and the like, and more specific examples are lactose, maltose, trehalose and the like. Examples of the sugar alcohol are mannitol, hydrogenated maltose starch syrup, maltitol, maltol, lactitol, xylitol, sorbitol, erythritol and the like. The saccharide or the sugar alcohol may be optionally selected from one or, two or more members of the aforementioned examples depending upon type, compounding ratio, content, etc. of an amino acid. The saccharide or the sugar alcohol may be contained, either alone or in combination, in an amount of preferably about 15 to 99% by weight, more preferably about 15 to 60% by weight and, still more preferably, about 20 to 40% by weight of the tablet.

Although there is no particular limitation on the average particle size of the saccharide, it is preferably 200 µm or smaller, more preferably 100 µm or smaller and particularly preferably 50 µm or smaller.

Such saccharides are preferably used after subjected to only mixing so that they do not coat the surface of the amino acid particles or amino acid-containing granules.

In the chewable tablet of the present invention, oral disintegration property is further improved when a sugar alcohol having good water solubility such as sorbitol, erythritol, xylitol and lactitol is contained in an amount of preferably 15% or more, more preferably 20% or more by weight of the tablet.

In the chewable tablet of the present invention, a hygroscopic amino acid such as proline, glycine, arginine and serine, and a sugar alcohol having good water solubility such as sorbitol, erythritol, xylitol and lactitol may be combined to improve the oral disintegration property. In that case, it is not always necessary to add the aforementioned oral disintegration promoting agent.

In the chewable tablet of the present invention, binders, lubricants and other additive components may be contained, if necessary, in addition to the aforementioned oral disintegration promoting agent.

Examples of the binder are polyvinylpyrrolidone, pullulan, acrylate-type polylmer, polyvinyl alcohol, gelatin, agar, acacia, powdered acacia, partly pregelatinized starch, macrogol and the like, and one or, two or more members of which may be used depending on need. The binder may be contained in an amount of preferably about 0.5 to 5% by weight, more preferably about 0.5 to 3% by weight and, still more preferably, about 0.5 to 2% by weight of the tablet. The binder may be used by dissolving in an aqueous solution such as water.

Although there is no particular limitation on the average particle size of the binder, it is preferably 200 µm or smaller, more preferably 100 µm or smaller and particularly preferably 50 µm or smaller.

Examples of the lubricant are sucrose esters of fatty acid, magnesium stearate, calcium stearate, sodium stearyl fumarate, talc, sodium laurylsulfate, light anhydrous silicic acid, hydrated silicon dioxide, sucrose esters of fatty acid and the like, and one or, two or more members of which may be used depending on need. The lubricant may be contained in an amount of preferably about 0.05 to 10% by weight, more preferably about 0.1 to 5% by weight and, still more preferably, about 0.1 to 3% by weight of the tablet.

Although there is no particular limitation on the average particle size of the lubricant, it is preferably 200 µm or smaller, more preferably 100 µm or smaller and particularly preferably 50 µm or smaller.

Such lubricants are preferably used after subjected to only mixing so that they do not coat the surface of the amino acid particles or amino acid-containing granules.

Examples of other additive component are corrigent for bitter taste which is a carbohydrate such as dextrin, starch, and cyclodextrin; β-carotene; food dyes such as Food Yellow No. 5, Food Red No. 2 and Food Blue No. 2; coloring agents such as food lake dyes, red iron oxide and niacin; vitamins such as vitamin E, ascorbic acid, vitamin B compounds, vitamin A and vitamin D, and derivatives thereof; minerals such as sodium; sweeteners such as aspartame, glucose, fructose, sucralose, stevia, saccharide, saccharin sodium and thaumatin; glidants such as fine silicon dioxide, calcium silicate, synthetic aluminum silicate and talc; foaming agents such as sodium bicarbonate; acid such as citric acid, malic acid and tartaric acid; flavors such as lemon, lemon lime, orange and menthol; cellulose or its derivatives; crystalline cellulose; microcrystalline cellulose; and the like, and one or, two or more members of which may be used depending on need. Other additive component as such may be contained in an amount of preferably about 0.01 to 5% by weight, more preferably about 0.1 to 3% by weight and, still more preferably, about 0.1 to 1% by weight of the tablet.

In the present invention, a chewable tablet refers to a preparation which is designed with a prerequisite of being chewed in the oral cavity and an example thereof is a masticatable tablet. The tablet size (diameter) of the chewable tablet is preferably about 7 to 20 mm, more preferably about 9 to 17 mm and, still more preferably, about 10 to 16 mm, while its tablet weight is preferably about 300 mg to 1.5 g, more preferably about 400 mg to 1 g and, still more preferably, about 500 mg to 900 mg. It is also possible in the chewable tablet of the present invention to increase the amount of an amino acid and, in that case, the weight of the tablet relative to the content of amino acid may be reduced. As a result, the thickness of the tablet relative to the diameter of the tablet can be reduced. The thickness of the chewable table of the present invention is preferably 2 to 8 mm, more preferably 3 to 6 mm and, particularly preferably, 3.5 to 5 mm.

As a result of making the tablet size smaller, it is now possible to place the chewable tablets having a higher amino acid content in a small portable container, which makes it possible to provide a packed portable product in which chewable tablets having a higher amino acid content are contained.

Examples of the packed portable product of the present invention are that where the chewable tablets of the present invention containing 35 to 70% by weight or, more preferably, 40 to 65% by weight of an amino acid having a thickness of 3 to 6 mm or, more preferably, 3.5 to 5 mm and diameter of 9 to 17 mm or, more preferably, 10 to 16 mm are placed in 7 to 24 tablets per line in a longitudinal direction and 2 to 5 lines in a transverse direction in a portable rectangular container of which inner wall has a height of 9 to 18 mm, a length of 48 to 72 mm and a width of 30 to 50 mm and that where 8 to 60 tablets are placed in a portable rectangular or elliptical container of which inner wall has a height of 40 to 70 mm, a maximum longitudinal width of 30 to 50 mm and a maximum transverse width of 9 to 30 mm, and the like. A desiccant which is commonly used in food may be stored together in the container. It is also possible that a desiccant and tablets are partitioned in the container.

The chewable tablet of the present invention is preferably softer than common tablets, and its hardness is preferably about 50 N or more, more preferably about 60 N or more, still more preferably about 65 N or more and, most preferably, about 70 N or more. The upper limit of the tablet hardness is usually about 120 N. The hardness is measured in the diameter direction of the tablet using a hardness tester manufactured by Japan Machinery (Type PTB-301).

Disintegration time for the tablet is preferably about 60 to 150 seconds, more preferably about 50 to 120 seconds and, still more preferably, about 40 to 100 seconds. With regard to the disintegration time for the tablet, a time period from when each of five healthy adults holds one tablet in the oral cavity until the tablet is disintegrated by saliva alone after chewing is measured and its mean value is adopted.

In the present invention, the powder particles comprising an amino acid and an oral disintegration promoting agent may comprise the aforementioned saccharide and/or sugar alcohol, binder, lubricant or commonly used other additives such as corrigent for bitter taste, coloring agent, sweetener, acid and flavor, alone or in combination, in addition to the amino acid and oral disintegration promoting agent.

An example of the method for manufacturing the chewable tablet of the present invention is a method in which powder particles comprising an amino acid and an oral disintegration promoting agent are subjected to compression molding, and the like.

There is no particular limitation on the compression molding method, and conventionally known methods may be used. Examples of such method include a common method in which a lubricant is added to powder particles comprising an amino acid and various additives such as an oral disintegration promoting agent, followed by mixing and compression molding, and a method in which a lubricant is previously applied on the surface of punch and on the die wall and then the powder particles are compression molded, and the like.

There is no particular limitation on the method for the preparation of such molded products, and examples thereof include a direct tableting method in which various materials are mixed and subjected to compression molding and a method in which all or a part of the materials are subjected to wet granulation or dry granulation, followed by compression molding.

An example of the wet granulation is a method in which a saccharide or a sugar alcohol is added to powder particles comprising an amino acid and an oral disintegration promoting agent, and water is added to the resulting mixture, followed by kneading and granulating. After that, a lubricant, an additive, etc. are added thereto and then subjected to compression molding, whereupon the tablets can be manufactured. The water includes, for example, purified water and the like, and there is no particular limitation on water provided that it is acceptable in view of the Food Sanitation Law or the Pharmaceutical Affairs Law of Japan. If necessary, a sugar, a sugar alcohol, a binder, etc. may be added to water.

The amount of water may be controlled upon addition of water to the material components or a mixture thereof. There is no particular limitation on a method of addition of water. Water may be added at a time, added dropwise or by spraying. There is no particular limitation on a method for spraying so far as it is a spraying method which is commonly used in the method for manufacturing preparations, and its examples include spray coating and spray dry, to be more specific, spraying with the use of a fluidized bed granulator and spraying with the use of a spray dryer. A mixture containing water is usually kneaded before being made into tablets. In the kneading of the mixture containing water, it is possible to use methods and apparatuses which is a commonly used means for manufacturing tablets. It is preferable that the resulting tablets are further dried. Drying may be carried out by any method which is commonly used a means for manufacturing preparations, such as vacuum dry, freeze dry and natural dry.

In an example of the dry granulation, a saccharide or a sugar alcohol is added to powder particles comprising an amino acid and an oral disintegration promoting agent; high compression is applied to the resulting mixture in a dry state for agglomeration; the bulks are ground; and the resulting powder particles are subjected to compression molding (a lubricant, an additive, etc. may be added at this stage) to prepare tablets.

There is no particular limitation on the apparatus used for granulation, and examples thereof include a agitation granulator, a high-speed agitation granulator, a fluidized bed granulator dryer, an extrusion granulator and a tumbling fluidized bed granulator dryer.

There is no particular limitation on the apparatus used for tableting, and an apparatus which is commonly used for molding or granulation of tablets may be used. For example, a rotary tableting machine, a single-shot tableting machine, etc. may be used.

### Examples

The following Examples are merely preferred embodiments of the present invention and are not intented to limit the technical scope of the present invention. With regard to the disintegration time for the tablet in the oral cavity, the time when each of five healthy adults holds one tablet in the oral cavity until the tablet is disintegrated after chewing by saliva alone is measured, and its mean value is adopted.

### <Example 1>

Mannitol(1,375 g), calcium carboxymethylcellulose(100 g), sucrose esters of fatty acid(50 g), citric acid(350 g) and flavor(125 g) were added to and mixed with a mixture of sieved amino acid nutritious components comprising leucine(1,200 g), isoleucine(600 g), valine(600 g) and glutamine(600 g).

After that, the above mixture was subjected to compression molding with tableting pressure of 20 kN using a rotary tableting machine (trade name: type AP-15; manufactured by Hata Tekkosho) equipped with a plane punch of 13 mm diameter to prepare chewable tablets (hereinafter, referred to as tablet 1), each weighing 500 mg (containing 300 mg of amino acids; amino acid content: about 60% by weight). As to the resulting tablet 1, tablet hardness was about 70 N and disintegration time of the tablet in the oral cavity was about 90 seconds and it was a tablet having an excellent mouthfeel as a chewable tablet. During the tableting step, no tableting trouble such as capping and sticking was observed.

### <Example 2>

Replacing mannitol in Example 1 with xylitol, tablets were manufactured. In the resulting tablet (hereinafter, referred to as tablet 2), tablet hardness was about 75 N and disintegration time of the tablet in the oral cavity was about 115 seconds, and it was a tablet having an excellent mouthfeel as a chewable tablet. During the tableting step, no tableting trouble such as capping and sticking was observed.

### <Example 3>

A mixture of sieved amino acid nutritious components comprising leucine(1,200 g), isoleucine(600 g), valine(600 g) and glutamine(600 g) was compounded with xylitol(1,375 g) and calcium carboxymethylcellulose(100 g), placed in a agitation granulator (trade name: Vertical Granulator VG-25; manufactured by Powlec) and mixed until the compounded substance became homogeneous to give a mixture (a).

Purified water was poured into a agitation granulator (trade name: Vertical Granulator VG-25; manufactured by Powlec) and kneaded and granulated for about 3 minutes with the mixture (a) and the resulting granules were taken out from the granulator and dried for 20 minutes at temperature of an intake air of not higher than 80°C using a fluidized bed dryer (manufactured by Gratt; type WSG-5). To 3,580 g of the resulting dry granules were added sucrose esters of fatty acid(40 g), citric acid(280 g) and flavor(100 g) to prepare a mixture for preparing tablets.

After that, the above mixture was subjected to compression molding with tableting pressure of 20 kN using a rotary tableting machine (trade name: type AP-15; manufactured by Hata Tekkosho) equipped with a plane punch of 15 mm diameter to prepare chewable tablets (hereinafter, referred to as tablet 3), each weighing 833 mg (containing 500 mg of amino acids; amino acid content: about 60% by weight). As to the resulting tablet 3, tablet hardness was about 71 N and disintegration time of the tablet in the oral cavity was about 120 seconds, and it was a tablet having an excellent mouthfeel as a chewable tablet. During the tableting step, no tableting trouble such as capping and sticking was observed.

### <Example 4>

Xylitol(1,375 g), calcium carboxymethylcellulose(100 g), citric acid(350 g) and flavor(125 g) were added to and mixed with a mixture of sieved amino acid nutritious components comprising leucine(1,200 g), isoleucine(600 g), valine(600 g) and glutamine(600 g).

After that, a rotary tableting machine (trade name: type AP-15; manufactured by Hata Tekkosho) equipped with a plane punch of 13 mm diameter was used and, before charging the above-prepared mixture in a die, sucrose esters of fatty acid as a lubricant is applied on the surfaces of upper and lower punches and on the die wall of the rotary tableting machine, and the mixture was subjected to compression molding with tableting pressure of 17 kN to prepare chewable tablets (hereinafter, referred to as tablet 4), each tablet weighing 500 mg (containing 333 mg of amino acids; amino acid content: about 60% by weight). As to the resulting tablet 4, tablet hardness was about 81 N and disintegration time of the tablet in the oral cavity was about 84 seconds, and it was a tablet having an excellent mouthfeel as a chewable tablet. During the tableting step, no tableting trouble such as capping and sticking was observed.

### <Example 5>

Chewable tablets each weighing 833 mg (amount of amino acids: 500 mg; amino acid content: about 60% by weight) were manufactured (hereinafter, referred to as tablet 5) in the same manner as in Example 3, except that Xylitol was replaced by hydrogenated maltose starch syrup. As to the resulting tablet 5, tablet hardness was about 68 N and disintegration time of the tablet in the oral cavity was about 114 seconds, and it was a tablet having an excellent mouthfeel as a chewable tablet. During the tableting step, no tableting trouble such as capping and sticking was observed.

### <Example 6>

A mixture of sieved amino acid nutritious components comprising leucine(1,200 g), isoleucine(600 g), valine(600 g) and glutamine(600 g) was compounded with trehalose(1,375 g) and sodium starch glycolate(100 g), placed in a fluidized bed granulator dryer (type WSG-5; manufactured by Gratt) and a binder solution where maltose(100 g) and pullulan(50 g) were dissolved in purified water(1,500 g) was sprayed thereon followed by drying to give a granulated dry product. To 3,660 g of the resulting granulated dry product were added sucrose esters of fatty acid(40 g), citric acid(200 g) and flavor (100 g)to prepare a mixture for preparing tablets.

After that, the above mixture was subjected to compression molding with tableting pressure of 20 kN using a rotary tableting machine (trade name: type AP-15; manufactured by Hata Tekkosho) equipped with a plane punch of 15 mm diameter to prepare chewable tablets (hereinafter, referred to as tablet 6), each weighing 833 mg (containing 500 mg of amino acids; amino acid content: about 60% by weight). As to the resulting tablet 6, tablet hardness was about 85 N and disintegration time of the tablet in the oral cavity was about 115 seconds, and it was a tablet having an excellent mouthfeel as a chewable tablet. During the tableting step, no tableting trouble such as capping and sticking was observed.

### <Example 7>

A mixture of sieved amino acid nutritious components comprising leucine(1,200 g), isoleucine(600 g), valine(600 g) and glutamine(600 g) was compounded with erythritol(1,375 g) and calcium carboxymethylcellulose(100 g), placed in a agitation granulator (trade name: Vertical Granulator VG-25; manufactured by Powlec) and mixed until the compounded substances became homogeneous to give a mixture (b).

Purified water to which pullulan(50 g) was added was poured into a agitation granulator (trade name: Vertical Granulator VG-25; manufactured by Powlec) and kneaded and granulated for about 3 minutes with the mixture (b), and the resulting granules were taken out from the granulator and dried for 20 minutes at temperature of an intake air of not higher than 80°C using a fluidized bed dryer (manufactured by Gratt; type WSG-5). To 3 , 580 g of the resulting dry granules were added sucrose esters of fatty acid(40 g) and flavor(100 g) to prepare a mixture for preparing tablets.

After that, the above mixture was subjected to compression molding with tableting pressure of 20 kN using a rotary tableting machine (trade name: type AP-15; manufactured by Hata Tekkosho) equipped with a plane punch of 15 mm diameter to prepare chewable tablets (hereinafter, referred to as tablet 7), each tablet weighing 833 mg (containing 500 mg of amino acids; amino acid content: about 60% by weight). As to the resulting tablet 7, tablet hardness was about 73 N and disintegration time of the tablet in the oral cavity was about 120 seconds, and it was a tablet having an excellent mouthfeel as a chewable tablet. During the tableting step, no tableting trouble such as capping and sticking was observed.

### <Example 8>

A mixture of sieved amino acid nutritious components comprising leucine(1,200 g), isoleucine(600 g), valine(600 g) and glutamine (600 g) was compounded with lactitol(1,375 g) and calcium carboxymethylcellulose(100 g), placed in a agitation granulator (trade name: Vertical Granulator VG-25; manufactured by Powlec) and mixed until the compounded substance became homogeneous to give a mixture (c).

Purified water was poured into a agitation granulator (trade name: Vertical Granulator VG-25; manufactured by Powlec) and kneaded and granulated for about 3 minutes with the mixture (c), and the resulting granules were taken out from the granulator and dried for 20 minutes at temperature of an intake air of not higher than 80°C using a fluidized bed dryer (manufactured by Gratt; type WSG-5). To 3,580 g of the resulting dry granules were added sucrose esters of fatty acid(40 g), citric acid(280 g) and flavor(100 g) to prepare a mixture for preparing tablets.

After that, the above mixture was subjected to compression molding with tableting pressure of 20 kN using a rotary tableting machine (trade name: type AP-15; manufactured by Hata Tekkosho) equipped with a plane punch of 15 mm diameter to prepare chewable tablets (hereinafter, referred to as tablet 8), each weighing 833 mg (containing 500 mg of amino acids; amino acid content: about 60% by weight). As to the resulting tablet 8, tablet hardness was about 65 N and disintegration time of the tablet in the oral cavity was about 120 seconds, and it was a tablet having an excellent mouthfeel as a chewable tablet. During the tableting step, no tableting trouble such as capping and sticking was observed.

### <Example 9>

A mixture of sieved amino acid nutritious components comprising leucine(1,200 g), isoleucine(600 g), valine(600 g) and glutamine(600 g) was compounded with sorbitol(1,375 g) and calcium carboxymethylcellulose(100 g), placed in a agitation granulator (trade name: Vertical Granulator VG-25; manufactured by Powlec) and mixed until the compounded substances became homogeneous to give a mixture (d).

Purified water was poured into a agitation granulator (trade name: Vertical Granulator VG-25; manufactured by Powlec) and kneaded and granulated for about 3 minutes with the mixture (d) and the resulting granules were taken out from the granulator and dried for 20 minutes at temperature of an intake air of not higher than 80°C using a fluidized bed dryer (manufactured by Gratt; type WSG-5). To 3,580 g of the resulting dry granules were added sucrose esters of fatty acid(40 g), citric acid(280 g) and flavor(100 g) to prepare a mixture for preparing tablets.

After that, the above mixture was subjected to compression molding with tableting pressure of 20 kN using a rotary tableting machine (trade name: type AP-15; manufactured by Hata Tekkosho) equipped with a plane punch of 15 mm diameter to prepare chewable tablets (hereinafter, referred to as tablet 9), each weighing 833 mg (containing 500 mg of amino acids; amino acid content: about 60% by weight). As to the resulting tablet 9, tablet hardness was about 71 N and disintegration time of the tablet in the oral cavity was about 120 seconds, and it was a tablet having an excellent mouthfeel as a chewable tablet. During the tableting step, no tableting trouble such as capping and sticking was observed.

### <Example 10>

A mixture of sieved amino acid nutritious components comprising leucine(1,200 g), isoleucine(600 g), valine(600 g) and glutamine(600 g) was compounded with erythritol(900 g) and calcium carboxymethylcellulose(100 g), placed in a agitation granulator (trade name: Vertical Granulator VG-25; manufactured by Powlec) and mixed until the compounded substances became homogeneous to give a mixture (e).

Purified water in which erythritol(500 g) and pullulan(50 g) were dissolved was poured as a binding solution into an agitation granulator (trade name: Vertical Granulator VG-25; manufactured by Powlec) and kneaded and granulated for about 3 minutes with the mixture (e). The resulting granules were taken out from the granulator and dried for 20 minutes at an intake air temperature of not higher than 80°C using a fluidized bed dryer (manufactured by Gratt; type WSG-5). To 3,580 g of the resulting dry granules were added sucrose esters of fatty acid(40 g), and flavor(100 g) to prepare a mixture for preparing tablets.

After that, the above mixture was subjected to compression molding with tableting pressure of 20 kN using a rotary tableting machine (trade name: type AP-15; manufactured by Hata Tekkosho) equipped with a plane punch of 15 mm diameter to prepare chewable tablets (hereinafter, referred as to tablet 10), each weighing 833 mg (containing 500 mg of amino acids; amino acid content: about 60% by weight). As to the resulting tablet 10 (flat tablet having 15 mm diameter and 4 mm thickness), tablet hardness was about 75 N and disintegration time of the tablet in the oral cavity was about 115 seconds, and it was a tablet having an excellent mouthfeel as a chewable tablet. During the tableting step, no tableting trouble such as capping and sticking was observed.

### <Example 11>

A mixture of sieved amino acid nutritious components comprising leucine(600 g), isoleucine(300 g), valine(300 g) and whey peptide hydrolysate(2,400 g) (manufactured by Meiji Dairies) was compounded with erythritol(900 g) and calcium carboxymethylcellulose(100 g), placed in a agitation granulator (trade name: Vertical Granulator VG-25; manufactured by Powlec) and mixed until the compounded substances became homogeneous to give a mixture (f).

Purified water in which erythritol(500 g) and pullulan(50 g) were dissolved was poured as a binding solution into a agitation granulator (trade name: Vertical Granulator VG-25; manufactured by Powlec) and kneaded and granulated for about 3 minutes with the mixture(f) and the resulting granules were taken out from the granulator and dried for 20 minutes at an intake air temperature of not higher than 80°C using a fluidized bed dryer (manufactured by Gratt; type WSG-5). To 3,580 g of the resulting dry granules were added sucrose esters of fatty acid(40 g), citric acid(280 g) and flavor(100 g) to prepare a mixture for preparing tablets.

After that, the above mixture was subjected to compression molding with tableting pressure of 20 kN using a rotary tableting machine (trade name: type AP-15; manufactured by Hata Tekkosho) equipped with a plane punch of 15 mm diameter to prepare chewable tablets (hereinafter, referred to as tablet 11), each tablet weighing 833 mg (containing 500 mg of amino acids; amino acid content: about 60% by weight). As to the resulting tablet 11 (flat tablet having 15 mm diameter and 4 mm thickness), tablet hardness was about 77 N and disintegration time of the tablet in the oral cavity was about 120 seconds, and it was a tablet having an excellent mouthfeel as a chewable tablet. During the tableting step, no tableting trouble such as capping and sticking was observed.

### <Test Example 1>

Tablet characteristics of the tablets 1 to 11 prepared hereinabove were compared with known amino acid-containing chewable tablets. In the known chewable tablet (a) where an emulsifying agent such as sucrose esters of fatty acid was added to 20% by weight of amino acids such as branched amino acids (2:1:1 (by weight) mixture of leucine, valine and isoleucine) and 72% by weight of hydrogenated maltose starch syrup, a molding property for tableting was bad and, after tableting, scratches and chips due to sticking were resulted in all of tablets. In general, when amount of main ingredient is increased, troubles upon tableting such as sticking and capping are apt to be generated but, in the tablets 1 to 11 of the present invention where amino acid content was made 3-fold of that of the chewable tablet (a), scratches and the like of the tablet due to sticking were not generated at all. In the chewable tablet (a), the disintegration time in the oral cavity was as long as 3 minutes or longer whereby its mouthfeel was not good as well.

Capping was generated in the known chewable tablet (b) wherein an emulsifier such as sucrose esters of fatty acid was added to 30% of branched amino acids (2:1:1 (by weight) mixture of leucine, valine and isoleucine) and 57% of starch.

On the contrary, in the tablets 1 to 11 of the present invention where amino acid content was made 2-fold of that of the chewable tablet (b), scratches and the like of the tablet due to capping were not generated at all. Further, although the chewable tablet (b) remained unpleasant feel after chewing in the oral cavity and thus was not preferred as a chewable tablet, the tablets 1 to 11 of the present invention were quickly dissolved in the oral cavity giving a favorable palatability.

Generally, with regard to amino acids such as branched amino acids to be used for sports and proline, etc. to be used for dieting, it is necessary to supply them in an amount of about 2 to 3 g at a time. With this respect, in the case of the chewable tablet (a), the amino acid content is as small as 200 mg in spite of the fact that it is a big tablet where the weight is 1 g and, in order to take the necessary amount, 10 to 15 tablets are necessary which that is not practical.

The chewable tablet (b) is a big tablet of 2 cm diameter and 2 g weight containing 600 mg of amino acids but, since the tablet is too big, it is not convenient for carrying.

On the contrary, in the tablets 3, 5 and 6 to 11 of the present invention, it is possible that 500 mg of amino acids are contained therein whereby the function can be achieved by administration of 5 to 6 tablets a day. In addition, since their weight is about one-third of the chewable tablet (b), they are convenient to carry. Moreover, there is no problem to the stability because the tablets of the present invention did not change color even when preserved for 3 months at 40°C in moisture conditions. Further, in a transportation test, their preserving property is remarkably good, because broken chips of the tablets are very little.

### <Test Example 2>

When the tablets 10 prepared in Example 10 (each being a flat tablet of 15 mm diameter and 4 mm thickness) were placed in two rows in a drawer type small portable container of 16 mm inner wall height, 56 mm length and 34 mm width together with a cylindrical drying agent of 7 mm thickness and 16 mm diameter, 20 tablets (total amount of the amino acids: 10 g) could be placed.

However, in the case of conventional capsules each containing 450 mg of amino acids (total amount of amino acids in 22 tablets: 10 g) (trade name: "BCAA'S" manufactured by Ultimate), they could not be placed even in a wide-caliber bottle of 38 mm diameter and 70 mm height which has been conventionally used as a container for pharmaceuticals for home use.

### <Test Example 3>

When the tablets 10 prepared in Example 10 (flat tablet of 15 mm diameter and 4 mm thickness) were placed in a portable container having elliptic bottom (having 34 mm maximum length and 20 mm maximum width for inner wall) with 50 mm inner wall height together with a drying agent (7 mm thickness and 16 mm diameter), 12 tablets could be placed.

### Industrial Applicability

In accordance with the present invention, there is provided a chewable tablet comprising an amino acid and having an improved oral disintegration property. In the chewable tablet, the amino acid content can be increased and the oral disintegration property is improved and, therefore, it has properties of good mouthfeel and easy taking.

## Claims

1. A chewable tablet comprising an amino acid and having an improved oral disintegration property.

2. An intraorally rapidly disintegrable chewable tablet comprising an amino acid.

3. The chewable tablet according to Claim 1 or 2, which comprises an oral disintegration promoting agent.

4. The chewable tablet according to any one of Claims 1 to 3, which further comprises a saccharide and/or a sugar alcohol.

5. A chewable tablet comprising an amino acid and an oral disintegration promoting agent.

6. The chewable tablet according to Claim 5, which further comprises a saccharide and/or a sugar alcohol.

7. The chewable tablet according to any one of Claims 3 to 6, wherein the oral disintegration promoting agent is sodium starch glycolate or calcium carboxymethylcellulose.

8. The chewable tablet according to any one of Claims 1 to 7, wherein the time until one tablet is disintegrated by saliva alone after chewing in the oral cavity of a healthy adult is 60 to 150 seconds.

9. The chewable tablet according to any one of Claims 1 to 8, wherein the amino acid content is 30 to 85% by weight.

10. The chewable tablet according to any one of Claims 1 to 9, wherein the hardness of the tablet is 60 N or more.

11. The chewable tablet according to any one of Claims 1 to 10, wherein the amino acid comprises one or, two or more members selected from the group consisting of valine, leucine and isoleucine.

12. The chewable tablet according to any one of Claims 1 to 11, wherein the amino acid is a mixture of a pure amino acid and a proteolytic mixture.

13. A method for manufacturing a chewable tablet having an improved oral disintegration property, which comprises subjecting powder particles comprising an amino acid and an oral disintegration promoting agent to compression molding.

14. A method for manufacturing an intraorally rapidly disintegrable chewable tablet, which comprises subjecting powder particles comprising an amino acid and an oral disintegration promoting agent to compression molding.

15. The method for manufacturing a chewable tablet according to Claim 13 or 14, wherein the powder particles further comprise a saccharide and/or a sugar alcohol.

16. A portable package in which the chewable tablet according to any one of Claims 1 to 12 is contained.

17. The portable package according to Claim 16, wherein the chewable tablet is contained together with a desiccant.
